# EUROPEAN PATENT APPLICATION

(11) **EP 3 460 801 A1**
(43) Date of publication of application: **27.03.2019**
(21) Application number: 17192144.8
(22) Date of filing: 20.09.2017
(51) Int. Cl.: G16H 10/00, G16H 20/00, G16H 40/00

(54) **PROVIDING SUBJECT-SPECIFIC INFORMATION**

(71) Applicant: Koninklijke Philips N.V., 5656 AE Eindhoven (NL)
(72) Inventor: SERLIE, Iwo Willem Oscar, 5656 AE Eindhoven (NL); PEETERS, Bob, 5656 AE Eindhoven (NL); MARTHERUS, Rudolph, 5656 AE Eindhoven (NL)
(74) Representative: de Haan, Poul Erik

(57) **Abstract**

Presented are concepts for providing subject-specific information. Once such concept comprises obtaining a subject-specific clinical model comprising a hierarchical representation of a plurality of clinical maps and clinical data relating to the subject, wherein the clinical data comprises information describing a time and a value of a property of each of a plurality of clinical events. A control signal for displaying clinical data relating to the subject is generated based on the subject-specific clinical model and a received input signal representative of at least one of: a selected time-window; and a selected clinical map of the subject-specific clinical model.

## Description

### FIELD OF THE INVENTION

This invention relates to the field of subject information provision and more particularly to the provision of subject-specific information from clinical maps.

### BACKGROUND OF THE INVENTION

Known systems for providing subject information (such as clinical data) use clinical models and maps that are hard-coded and static.

A clinical map typically comprises graphical information for graphically representing clinical data relating to a subject. A clinical map may therefore provide a graphical representation of a region of anatomy or pathology of a human or animal. For instance, a clinical map may provide information for graphically representing clinical data relating to a heart, but may also be structured in a hierarchical form so that a refinement (e.g. sub-level) of the clinical map of the heart comprises a clinical map of a heart valve of the heart (e.g. information for graphically representing clinical data relating to the heart valve).

For a subject having numerous medical events (e.g. a subject suffering from chronic diseases and/or regularly visiting a medical practitioner), the clinical data relating to the subject may be extensive and densely populated with respect to time. Exploration and/or analysis of the clinical data relating to such subject may therefore be resource intensive and/or difficult. Also, graphical or visual representation of the clinical data may be complex and/or difficult to understand.

Also, where there may be multiple interacting clinical issues, conventional systems are unable to automatically provide or display clinical information from multiple clinical maps in context (or in a manner which is quick and easy to interpret). With intuitive presentation and navigation of subject-specific clinical information being of paramount importance in Clinical Decision Support (CDS), there remains a need for approaches to providing subject-specific information a manner which is simple, intuitive and easy to navigate.

### SUMMARY OF THE INVENTION

The invention aims to at least partly fulfil the aforementioned needs. To this end, the invention provides devices, systems and methods as defined in the independent claims. The dependent claims provide advantageous embodiments.

There is provided a method for providing subject-specific information. The method comprises: obtaining a plurality of clinical maps associated with a subject, each clinical map comprising graphical information for graphically representing clinical data relating to the subject; obtaining clinical data relating to the subject, the clinical data comprising information describing a time and a value of a property of each of a plurality of clinical events; and generating a subject-specific clinical model based on the plurality of clinical maps and the obtained clinical data, the subject-specific clinical model comprising a hierarchical representation of the plurality of clinical maps.

Proposed is a concept of composing a subject-specific clinical model from multiple clinical maps which facilitates the visualisation of clinical events on a timeline. Combination of clinical data in a clinical model and relating the clinical data to time (e.g. using a timeline) helps a user to differentiate between a chronological and clinical context. Such proposals may increase the clinical value of information by enabling clinical information to be visualised (e.g. displayed or graphically represented) relative to each other and in a chronological and/or clinical context. This may support assessment, enable improved focus on a diagnosis, and/or support clinical planning. Improved CDS may therefore be provided by proposed concepts. Also, proposed embodiments may support visual navigation through subject-specific clinical (e.g. diagnostic) information with respect to time.

Proposed embodiments may provide a clinical model comprising structured health domain content that is specific to a subject. The provided clinical model may be structured in a hierarchy, thus expressing refinements of models according to clinical semantics (e.g. a heart valve model might be a refinement of a heart model). Such a clinical model may be generated from clinical maps, wherein a clinical map may be thought of a graphical interaction paradigm with structured clinical data. For instance, a clinical map may comprise a graphical representation of clinical data, or a definition thereof.

Accordingly, concepts are proposed which may enrich the display or visualisation of clinical information relating to a specific subject by creating a subject-specific clinical model using a plurality of clinical maps. The clinical maps may be combined according to available clinical information for the subject and facilitate visualisation of clinical information with respect to time (e.g. on a timeline).

A clinical map, as opposed to a clinical model, may provide or define a graphical representation of a clinical data (e.g. a problem with an anatomical feature of a subject, treatment method(s) for a subject, medical support for pathologies, etc.). Some proposed embodiments may therefore be thought of overlaying a standard clinical model (e.g. anatomical atlas or map representation of a person) with one or more graphical representations indicating unique pathologies, clinical problems or treatments relevant to a specific-subject. Presentation or visualisation of the clinical data may then be made dependent on a time (e.g. event time or time stamp) associated with the clinical data. For instance, a time window of interest may be defined and data relating to one or more clinical events having a time within the time window of interest may be displayed in a particular manner (e.g. unhidden, emphasised, highlighted, etc.). Similarly, data relating to clinical event having a time outside of the time window of interest may be displayed in an alternative manner (e.g. hidden, de-emphasized, etc.). Such display may be on a timeline, in the clinical model, or both, for example. A timeline and clinical map may thus display data relating to time-based clinical events within an indicated time segment, and this may be done in a synchronized manner. Embodiments may therefore provide a medical system that facilitates interaction with structured or annotated aggregated clinical data relating to a specific subject, and this may be done in a time-based context.

Advantages associated with proposed embodiments may therefore include: (a) localization of clinical information representations without software engineering efforts; (b) intuitive navigation through clinical information with respect to time; (c) increased clinical value of available information though the visualisation of clinical information elements relative to time and/or in a clinical context (e.g. supporting assessment, focussing on a diagnosis, supporting planning, etc.).

The subject-specific clinical model may be representative of an anatomical relationship between anatomical features of the subject. This may support the presentation and/or navigation of subject-specific clinical information in a manner which is representative a relative location and/or relationship in a subject's body. Simple and intuitive presentation or navigation of clinical information may thus be facilitated by proposed embodiments. For instance, by positioning clinical maps in a graphical representation of a subject's body according to the location of the associated anatomical feature(s), a clinical model may be provided which enables quick and easy navigation of the clinical maps.

The clinical data may further comprise information describing an anatomical feature associated with each of the plurality of clinical events. The step of generating a subject-specific clinical model may then comprise defining a hierarchical representation of the plurality of clinical maps based on the information describing an anatomical feature associated with each of the plurality of clinical events. In this way, a generated clinical model may summarize time-based events according to a detail level defined by the clinical model.

The step of generating a subject-specific clinical model may comprise: combining at least two of the plurality of clinical maps based on a blending function. By way of example, the blending function may be representative of at least one of: a definition of an instance of a clinical model; a hierarchy of clinical models; and a hierarchy of clinical maps. A clinical model may thus be amended with multiple clinical maps, and such clinical maps may be combined into the model in a hierarchical manner and/or a manner which is representative of an anatomical relationship or context. Here, it is noted that a clinical map be defined as a part of another clinical map (e.g. a lower-level abstraction or sub-portion). A clinical map may therefore be an abstraction of another clinical map. Where an abstract representation is superimposed, a blending function may be adapted to avoid anatomical inconsistencies and adhere to a required rendering size.

Embodiments may further comprise annotating the generated subject-specific clinical model based on the information describing a time of each of the plurality of clinical events. For instance, one or more graphical representations may overlay a clinical map in a graphical representation of generated model. This may facilitate quick, easy and intuitive navigation of the clinical model with respect to a time frame or time window of interest.

According to another aspect of the invention, there is provided a method for displaying subject-specific information. The method comprises: obtaining a subject-specific clinical model comprising a hierarchical representation of a plurality of clinical maps and clinical data relating to the subject, the clinical data comprising information describing a time and a value of a property of each of a plurality of clinical events; receiving an input signal representative of at least one of: a selected time-window; and a selected clinical map of the subject-specific clinical model; generating a control signal for displaying clinical data relating to the subject based on the subject-specific clinical model and the received input signal; and displaying clinical data relating to the subject in accordance with the generated control signal.

Proposed embodiments may thus help a user (such as a medical practitioner, doctor, surgeon, technician, etc.) to differentiate between a chronological and clinical context. The combination of time-based clinical data and clinical maps may enable a user to focus on part of the chronology and/or part of clinical context, thus reducing complexity or confusion that may otherwise be caused by presenting additional and/or irrelevant clinical data.

Embodiments may increase a clinical value of time-clustered events by using clinical criteria rather than technical criteria such as imaging modality procedure type.

In some embodiments, the input signal may be representative of a selected time-window. The step of generating a control signal for displaying clinical data relating to the subject may then comprise generating a control signal for controlling the display of clinical data based on whether a time of a clinical event matches the selected time-window. In this way, a time period of interest may be defined and used to control the display of clinical information. For instance, only clinical data relating to clinical events occurring within the time period of interest may be displayed, thereby preventing irrelevant and/or extraneous information from being presented.

In an embodiment, the input signal may be representative of a selected clinical map of the subject-specific clinical model. The step of generating a control signal for displaying clinical data relating to the subject may then comprise generating a control signal for controlling the display of clinical data based on whether a value of a property of a clinical event relates to the selected clinical map. In this way, a clinical map of interest may be defined and used to control the display of clinical information. For instance, only clinical data relating to clinical map of an anatomical feature of interest may be displayed, thereby preventing irrelevant and/or extraneous information from being presented.

According to another aspect, there is provided a computer program product for providing or displaying subject-specific information, wherein the computer program product comprises a computer-readable storage medium having computer-readable program code embodied therewith, the computer-readable program code configured to perform all of the steps of a proposed embodiment.

Thus, there is also provided a computer system comprising: a computer program product according to proposed embodiment; and one or more processors adapted to perform a method according to a propose concept by execution of the computer-readable program code of said computer program product.

According to another aspect of the invention, there is provided a system for providing subject-specific information. The system comprises: an input interface adapted to obtain a plurality of clinical maps associated with a subject, each clinical map comprising graphical information for graphically representing clinical data relating to the subject, and to obtain clinical data relating to the subject, the clinical data comprising information describing a time and a value of a property of each of a plurality of clinical events; and a processing module adapted to generate a subject-specific clinical model based on the plurality of clinical maps and the obtained clinical data, the subject-specific clinical model comprising a hierarchical representation of the plurality of clinical maps.

In some embodiments, the clinical data may further comprise information describing an anatomical feature associated with each of the plurality of clinical events. The processing module may then be adapted to define a hierarchical representation of the plurality of clinical maps based on the information describing an anatomical feature associated with each of the plurality of clinical events.

According to yet another aspect of the invention, there is provided a system for displaying subject-specific information. The system comprises: an interface adapted to obtain a subject-specific clinical model comprising a hierarchical representation of a plurality of clinical maps and clinical data relating to the subject, the clinical data comprising information describing a time and a value of a property of each of a plurality of clinical events; an input signal interface adapted to receive an input signal representative of at least one of: a selected time-window; and a selected clinical map of the subject-specific clinical model; an output interface adapted to generate a control signal for displaying clinical data relating to the subject based on the subject-specific clinical model and the received input signal; and a display unit adapted to display clinical data relating to the subject in accordance with the generated control signal.

The processing module may be remotely located from the display unit, and the control signal may thus be communicated to the display unit via a communication link. In this way, a user (such as a medical professional) may have an appropriately arranged display system that can receive and display subject-specific information at a location remotely located from the processing module. Embodiments may therefore enable a user to remotely review subject-specific information using a portable display device, such as a laptop, tablet computer, mobile phone, PDA, etc.

The system may further comprise: a server device comprising the data processing module; and a client device comprising the display unit. Dedicated data processing means may therefore be employed for the purpose of generating a subject-specific clinical model, and generating a control signal, thus reducing processing requirements or capabilities of other components or devices of the system.

The system may further comprise a client device, wherein the client device comprises the data processing module and the display unit. In other words, a user (such as a doctor or medical professional) may have an appropriately arranged client device (such as a laptop, tablet computer, mobile phone, PDA, etc.) which processes received data in order to generate a subject-specific clinical model and generate a control signal.

Thus, it will be understood that processing capabilities may therefore be distributed throughout the system in different ways according to predetermined constraints and/or availability of processing resources.

These and other aspects of the invention will be apparent from and elucidated with reference to the embodiment(s) described hereinafter.

### BRIEF DESCRIPTION OF THE DRAWINGS

Examples in accordance with aspects of the invention will now be described in detail with reference to the accompanying schematic drawings, in which:
Figure 1 is an exemplary flow diagram of a method for providing subject-specific information according to an embodiment;
Figure 2 is an exemplary flow diagram of a method for displaying subject-specific information according to an embodiment, wherein the method employs the method of Figure 1;
Figure 3A is an illustration summarising a system for displaying clinical data of time-based clinical events on a timeline and in a clinical model in accordance with proposed concepts;
Figure 3B depicts a modification to the embodiment of Figure 3A;
Figure 4 is a simplified block diagram of a system according to an embodiment; and
Figure 5 is a simplified block diagram of a computer within which one or more parts of an embodiment may be employed.

### DETAILED DESCRIPTION OF THE EMBODIMENTS

Proposed is a concept for enabling the provision of subject-specific clinical information in a visual form using clinical data maps. Clinical information relating to time-based clinical events may be provided in a clinical model and displayed on a timeline. The timeline and clinical model may be controlled and/or manipulated in conjunction with each other, thus enabling clinical information to be viewed in a chronological and/or clinical context.

Clinical data maps may be combined into a subject-specific clinical model taking account of time-based clinical events that are associated with available clinical information. A clinical model provided by such proposals may thus facilitate simple and intuitive navigation and assessment of subject-specific clinical information with reference to the time(s) at which the clinical information was obtained/provided, for example by enabling clinical information to be visualized and/or navigated in a chronological context. Improved CDS may therefore be provided by proposed embodiments.

Embodiments of the present invention are therefore directed toward enabling the presentation of subject-specific clinical information with reference to timing information so as to facilitate or enhance a CDS process. Further, embodiments may be aimed at enabling the display or visualisation of subject-specific clinical data or information with simplicity, accuracy and chronological context while providing enough detail so that a clinical assessment or decision is facilitated or supported (preferably with increased efficiency and/or reliability). A generated clinical model may be used to identify or indicate patterns in clinical data and/or clinical events, which may assist in clinical support assessment, medical diagnosis and/or support planning for example.

Embodiments are based on the insight that the display of clinical information may be enhanced by employing timing information of clinical data to create a subject-specific clinical model that can be combined (synchronised) with a timeline.

A clinical map, as opposed to a clinical model, may provide or define a graphical representation of a clinical data (e.g. a problem with an anatomical feature of a subject, treatment method(s) for a subject, medical support for pathologies, etc.).

A clinical model, on the other hand, may comprise structured health domain content that is specific to a subject. Thus, a clinical model may be structured in a hierarchy, thereby expressing refinements of models according to clinical semantics (e.g. a heart valve model might be a refinement of a heart model). Such a clinical model may be represented in the form of an anatomical atlas or map representation of a person, thus enabling visualisation of the anatomical features and available clinical data relating an anatomical feature.

According to proposed concepts, a clinical model may comprise clinical maps, wherein a clinical map may be thought of a graphical interaction paradigm with structured clinical data.

Some proposed embodiments may therefore be thought of combining clinical maps into a time-based clinical model with one or more graphical representations indicating unique pathologies, clinical problems or treatments relevant to a specific-subject. Thus, there may be provided a medical system that facilitates interaction with structured or annotated aggregated clinical data relating to a specific subject and which comprises associated timing information which facilities presentation of the clinical data with reference to time.

Illustrative embodiments may be utilized in many different types of clinical, medical or subject-related environments, such as a hospital, doctor's office, ward, care home, person's home, etc. In order to provide a context for the description of elements and functionality of the illustrative embodiments, the Figures are provided hereafter as examples of how aspects of the illustrative embodiments may be implemented. It should therefore be appreciated the Figures are only examples and are not intended to assert or imply any limitation with regard to the environments, systems or methods in which aspects or embodiments of the present invention may be implemented.

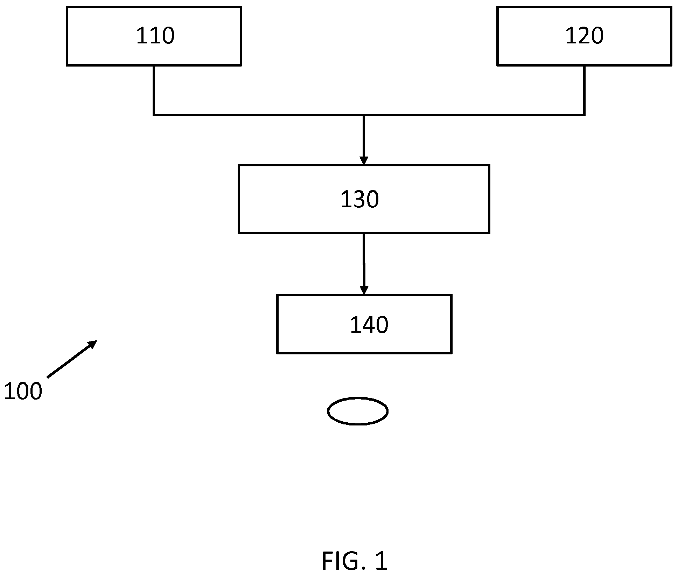

Referring now to Figure 1, there is depicted an exemplary flow diagram of a method 100 for providing subject-specific information according to an embodiment.

In step 110, a plurality of clinical maps associated with a subject is obtained. Each clinical map comprises graphical information for graphically representing clinical data relating to the subject. For example, a clinical map may focus on a specific anatomical 'system' such as the skeletal, circulatory, digestive, urinary, or nervous system. Thus, specific subject data might only relate to a specific system. For a specific subject, a clinical map of the heart might show a specific valve superimposed as another layer (e.g. in a combination of clinical maps). For another subject, a clinical map might show a nervous system superimposed on a heart based on a specific defect in the nervous system (e.g. an affected AV node). The combination of maps may thus be based on specific clinical data.

In step 120, clinical data relating to the subject is obtained. Here, the clinical data comprises information describing a time and a value of a property of each of a plurality of clinical events. For instance, clinical data obtained for a clinical event (such as medical assessment, monitoring instance, medical intervention instance, etc.) will include values of clinical parameters and associated timing information describing the time(s) at which the values were obtained (e.g. a detection timestamp).

Based on the plurality of clinical maps and the obtained clinical data, a subject-specific clinical model is generated in step 130. Here, the generated subject-specific clinical model comprises a hierarchical representation of the plurality of clinical maps. Further, the subject-specific clinical model is representative of an anatomical relationship between anatomical features of the subject.

By way of further example, the step 130 of generating a subject-specific clinical model can include combining at least two of the plurality of clinical maps based on a blending function. The blending function may be a function which is representative of at least one of: a definition of an instance of a clinical model; a hierarchy of clinical models; and a hierarchy of clinical maps.

Furthermore, as depicted in Figure 1, the proposed embodiment also includes the step 150 of annotating the generated subject-specific clinical model based on the information describing a time of each of the plurality of clinical events. For instance, one or more graphical representations may be overlaid on a clinical map in a graphical representation of the generated model. This can facilitate quick, easy and intuitive navigation of the clinical model.

It will be appreciated that execution of the method of Figure 1 provides a subject-specific clinical model comprising timing information for clinical data, and this may then be leveraged for the purpose of displaying subject-specific information to user (such as a medical professional for example) in a chronological context. Proposed concepts may therefore provide a method for displaying subject specific information which incorporates an embodiment of providing subject-specific information.

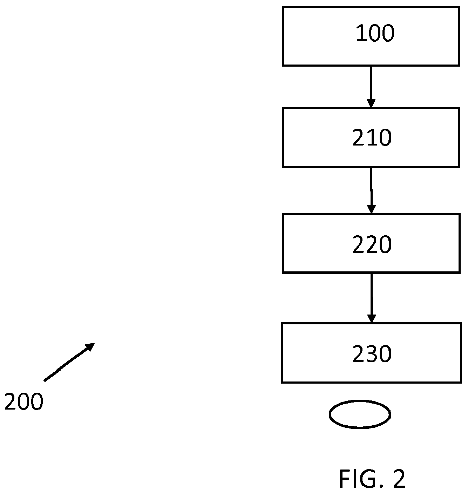

For example, one may consider interaction with a model provided in accordance with an embodiment. For instance, if a user selects a heart valve in a specific layer of the aggregation of clinical maps (e.g. a valve on a heart), events that are associated with the specific body part can be selected and identified (e.g. highlighted) on a timeline. Such events may, for example, include: a procedure in which the valve was treated; follow-up events; and/or planned follow-up events. Thus, a selection on a clinical map may result in showing an event on a timeline. If it is a planned event, there might be a planned action associated. Similarly, selection of an event in a timeline may result in the display of a subject-specific composed clinical map. By way of further example, the clinical map might also play as a movie (where the timeline functions as a time selection or control interface). By way of further example, Figure 2 depicts an exemplary flow diagram of a method 200 for displaying subject-specific information according to an embodiment, wherein the method 200 employs the method 100 of Figure 1.

More specifically, the method 200 begins with the step 100 of providing subject-specific information according to proposed embodiment of Figure 1. Execution of step 100 therefore results in the provision of a subject-specific clinical model comprising a hierarchical representation of a plurality of clinical maps and clinical data relating to the subject, wherein the clinical data comprises information describing a time and a value of a property of each of a plurality of clinical events.

In step 210, an input signal is received which is representative of a representative of at least one of: a selected time-window; and a selected clinical map of the subject-specific clinical model (from step 100). A control signal is then generated in step 220 based on the subject-specific clinical model and the received input signal, wherein the control signal is for controlling a display device to display clinical data relating to the subject.

Where the input signal is representative of a selected time-window, the step 220 of generating a control signal for displaying clinical data relating to the subject comprises generating a control signal for controlling the display of clinical data based on whether a time of a clinical event matches the selected time-window.

Where the input signal is representative of a selected clinical map of the subject-specific clinical model, the step 220 of generating a control signal for displaying clinical data relating to the subject comprises generating a control signal for controlling the display of clinical data based on whether a value of a property of a clinical event relates to the selected clinical map.

By way of further example, however, the control signal can also be generated based on a display position of the selected clinical map of the subject-specific clinical model. In this way, display considerations (such available display space, already-displayed graphical elements, overlapping content, etc.) may be accounted for so as to ensure correct or appropriate display instructions are provided by the control signal.

The clinical data relating to the subject is then displayed in step 230 in accordance with the generated control signal.

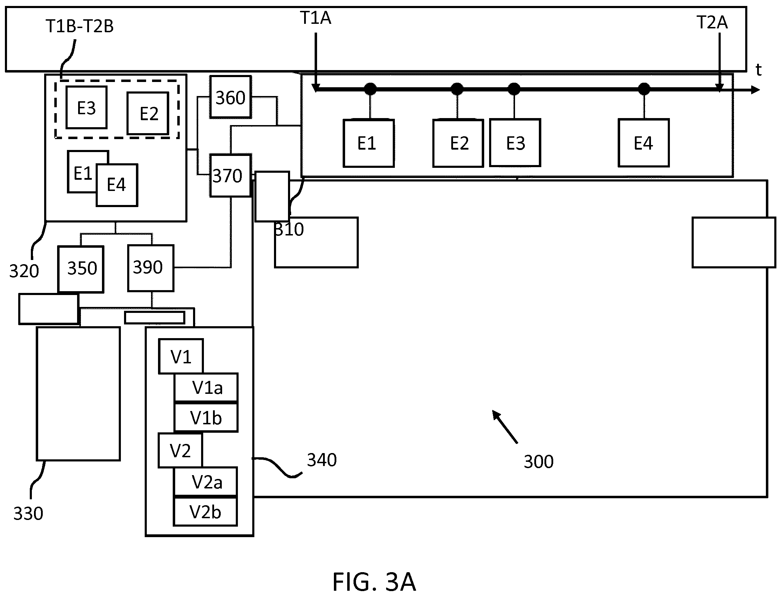

Referring now to Figure 3A, there is provided an illustration summarising a system 300 for displaying clinical data of time-based clinical events on a timeline and in a clinical model in accordance with proposed concepts. The system is for displaying time-based clinical events on a timeline 310 and on a clinical map 320. It is also adapted to enable manipulation of the display of such information in a synchronised manner.

A first timeline (or timeline component) 310 is displayed as a first instance of the timeline (with time t increasing from left to right) showing a first plurality of clinical events (E1, E2, E3, E4) in a time window bounded by a first start time T1A and a first end time T2A.

A clinical model 330 (comprising structured health domain content) describes semantics of the clinical events containing a time stamp and a body region identifier/descriptor.

A value set 340 describes a set of possible values (V1, V1a, V1b, V2, V2a, V2b) of the body region descriptor. The values are organized hierarchically (e.g. V1a and V1b depend from V1) allowing a value set designer to model semantic levels of body region descriptors.

The clinical map 320 represents a plurality of events in a first clinical map segment bounded by a second start time T1B and a second end time T2B. A location on a clinical map is bounded to a clinical model parameter (e.g. body region descriptor). In the depicted example, the second plurality of clinical events in the time window bounded by the second start time T1B and a second end time T2B comprise the clinical events E2 and E3 (as depicted by the dashed box labelled "T1B-T2B", and which may be thought of as a first region of interest).

These clinical maps 320 and the timeline 310 are arranged to be synchronized with each other such that, when a user provides an indication (via user input interface 350) to change the time window (e.g. by changing the first start time T1A and/or the first end time T2A), a timeline processing unit 360 determines an updated timeline segment and updated clinical map segment. In this way, the timeline 310 is arranged to be updated according to the new time segment/window of interest. Also, the clinical map 320 is arranged to be updated according to the new time segment/window of interest.

The system 300 thus synchronizes the timeline 310 with respect to the clinical map, wherein the user input interface 350 enables a user to indicate changes to regions and/or times of interest.

The system 300 also comprises a clinical segment updater 370 which is adapted to determine an updated clinical map segment according to the region of interest (e.g. the second start time T1B and a second end time T2B).

As a result, the timeline 310 and clinical map 320 are adapted to display time-based clinical events within a region and/or timeframe of interest. Thus, the clinical map 320 may be employed as a control panel for the timeline, since interaction with the clinical map may be used to control and alter a selection of relevant clinical data on the timeline. Similarly, the timeline may be employed as a control panel for the clinical map, since interaction with the timeline (e.g. modification of the timeframe of interest) may be used to control and alter a selection of clinical dap in the clinical map.

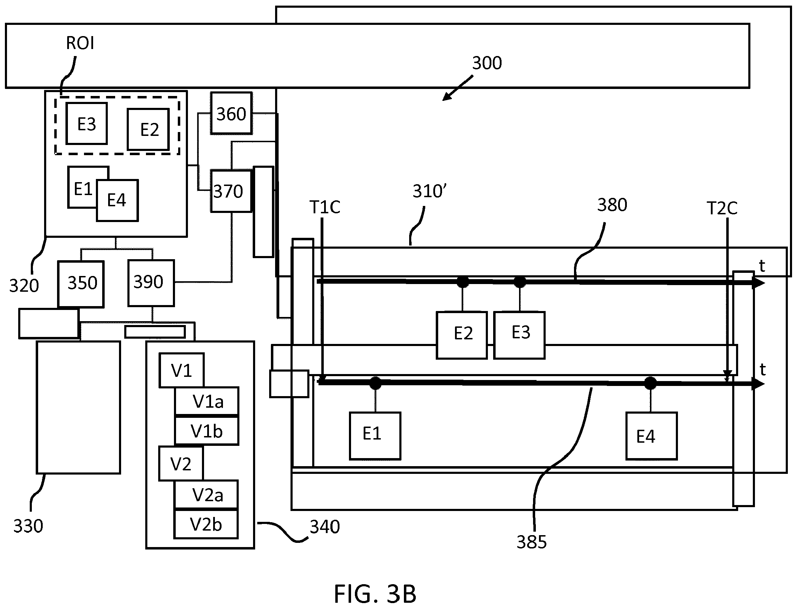

Furthermore, referring now to Figure 3B, an embodiment can separate a timeline into multiple segments 310A and 310B according to a region of interest in the clinical map 320.

A second timeline (or timeline component) 310' is displayed as a second instance of the timeline (with time t increasing from left to right) showing a first plurality of clinical events (E1, E2, E3, E4) in a time window bounded by a third start time T1C and a third end time T2C. Here, however, the second timeline 310' is adapted to be displayed in multiple segments, wherein each segment is associated with a respective region of interest of the clinical map 320.

In particular, the clinical segment updater 370 determines two timeline segments derived from a second timeline instance 310' according to the clinical map. Here, the clinical segment updater 370 groups time-based clinical events within the region of interest ROI of the clinical map 320 onto a first timeline segment 380 and groups time-based events outside the region of interest ROI of the clinical map 310 onto a second timeline segment 385. In this example, clinical events E2 and E3 are within the region of interest ROI of the clinical map 320 and so are provided on the first timeline segment 380. Also, clinical events E1 and E4 are outside the region of interest ROI of the clinical map 320 and so are provided on the second timeline segment 385. As a result, the second timeline instance 310' displays time-based events in synchronized segments according to the ROI defined in the clinical map 320.

Accordingly, it will be understood that the embodiment of Figure 3B is arranged to separate a timeline 310' into multiple segments according to a clinical model parameter (e.g. identical body region descriptor). For this purpose, the clinical segment updater 370 can be configured to determine multiple segments according to a grouping result of a clinical map grouping unit 390.

Embodiments may thus be adapted to dynamically segment a timeline into time-based segments where each segment is associated with a specific model descriptor at a specific semantical level.

By way of example, the clinical map grouping unit 390 is arranged to group multiple events according to a clinical model 330 with identical model parameter value 340 into a single derived event. For example, a body region descriptor value taken from a value set 390.

Also, the clinical map grouping unit 390 can group according to a semantic level (e.g. a hierarchical level in the value set 390). For example, grouping might be according to values 'v1' and 'v2' or according to values 'v1a', 'v1b', 'v2a', 'v2b'.

The clinical map 320 may be configured to display a derived event in a visually discriminating way so as to indicate a group. For instance, such a group can be summarized by a graphical symbol indicating the value of a parameter.

The user input interface 350 may also be configured to enable a user to select a derived event which may, in effect, select all underlying events.

It will therefore be appreciated that the clinical map 320 may be arranged to summarise time-based clinical events according to a detail level defined by a user input and/or clinical model 330. As a result, the timeline 310 and clinical map 320 units can display the time-based events within a selected time segment in a synchronized way. Also, embodiment may be adapted to display time-based events outside a selected time segment of interest in a visually different way (e.g. at a reduced size and/or increased transparency value).

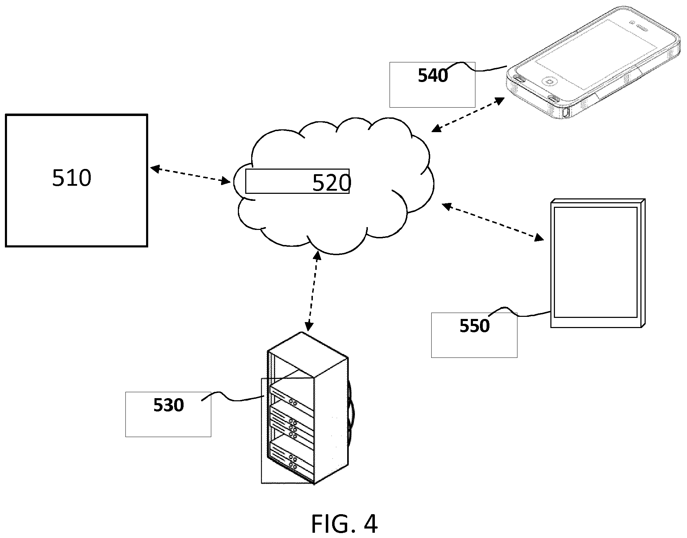

Referring now to Fig. 4, there is depicted an embodiment of a system according to an embodiment of the invention comprising an input system 510 arranged to obtain various sets of information

Here, the input system 510 is adapted to obtain a plurality of clinical maps associated with a subject, each clinical map comprising graphical information for graphically representing clinical data relating to the subject, and to obtain clinical data relating to the subject (the clinical data comprising information describing a time and a value of a property of each of a plurality of clinical events). The input system 510 is adapted to output one or more signals which are representative of obtained information/data.

The input system 510 communicates the output signals via the internet 520 (using a wired or wireless connection for example) to a remotely located data processing system 530 (such as server).

The data processing system 530 is adapted to receive the one or more output signals from the input system 510 and process the received signal(s) to generate a subject-specific clinical model, wherein the subject-specific clinical model comprises a hierarchical representation of a plurality of clinical maps. Thus, the data processing 530 provides a centrally accessible processing resource that can receive information from the input system 510 and run one or more algorithms to transform the received information into a subject-specific clinical model comprising timing information relating to a plurality of clinical events. Information relating to the subject-specific clinical model can be stored by the data processing system (for example, in a database) and provided to other components of the monitoring system. Such provision of information about a subject-specific clinical model may be undertaken in response to a receiving a request (via the internet 520 for example) and/or may be undertaken without request (i.e. 'pushed').

For the purpose of receiving information about a subject-specific clinical model from the data processing system 530, and thus to subject-specific information to be viewed, the system further comprises first 540 and second 550 mobile computing devices.

Here, the first mobile computing device 540 is a mobile telephone device (such as a smartphone) with a display for displaying clinical models, clinical maps and/or timelines in accordance with embodiments of the proposed concepts. The second mobile computing device 550 is a mobile computer such as a Laptop or Tablet computer with a display for displaying clinical models, clinical maps and/or timelines in accordance with embodiments of the proposed concepts.

The data processing system 530 is adapted to communicate clinical model output signals to the first 540 and second 550 mobile computing devices via the internet 520 (using a wired or wireless connection for example). As mentioned above, this may be undertaken in response to receiving a request from the first 540 or second 550 mobile computing devices.

Based on the received output signals, the first 540 and second 550 mobile computing devices are adapted to display one or more graphical elements in a display area provided by their respective display. For this purpose, the first 540 and second 550 mobile computing devices each comprise a software application for processing, decrypting and/or interpreting received clinical model output signals in order to determine how to display graphical elements. Thus, the first 540 and second 550 mobile computing devices each comprise a processing arrangement adapted to determine an attribute of clinical model, clinical map and/or clinical timeline, and to generate a display control signal for modifying at least one of the size, shape, position, orientation, pulsation or colour of a graphical element based on the determined attribute of the clinical model, clinical map and/or timeline.

The system can therefore communicate information about subject-specific clinical models, clinical maps and clinical timelines to users of the first 540 and second 550 mobile computing devices. For example, each of the first 540 and second 550 mobile computing devices may be used to display graphical elements to a medical practitioner, doctor, consultant, technician or caregiver for example.

Implementations of the system of Figure 4 may vary between: (i) a situation where the data processing system 530 communicates display-ready clinical data, which may for example comprise display data including graphical elements (e.g. in JPEG or other image formats) that are simply displayed to a user of a mobile computing device using conventional image or webpage display (can be web based browser etc.); to (ii) a situation where the data processing system 530 communicates raw data set information that the receiving mobile computing device then transforms to a clinical model or timelines, processes to determine one or more attributes of the clinical model or timeline, and then displays graphical elements based on the determined one or more attributes (for example, using local software running on the mobile computing device). Of course, in other implementations, the processing may be shared between the data processing system 530 and a receiving mobile computing device such that part of the clinical model or timeline generated at data processing system 530 is sent to the mobile computing device for further processing by local dedicated software of the mobile computing device. Embodiments may therefore employ server-side processing, client-side processing, or any combination thereof.

Further, where the data processing system 530 does not 'push' subject-specific clinical model information (e.g. clinical model output signals), but rather communicates information in response to receiving a request, the user of a device making such a request may be required to confirm or authenticate their identity and/or security credentials in order for information to be communicated.

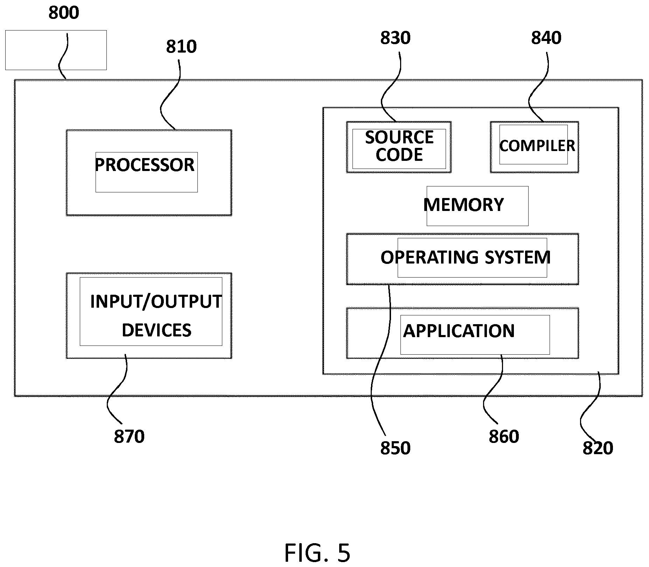

Figure 5 illustrates an example of a computer 800 within which one or more parts of an embodiment may be employed. Various operations discussed above may utilize the capabilities of the computer 800. For example, one or more parts of a system for providing subject-specific information (or display unit thereof) may be incorporated in any element, module, application, and/or component discussed herein.

The computer 800 includes, but is not limited to, PCs, workstations, laptops, PDAs, palm devices, servers, storages, and the like. Generally, in terms of hardware architecture, the computer 800 may include one or more processors 810, memory 820, and one or more I/O devices 870 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 840, source code 830, and one or more applications 860 in accordance with exemplary embodiments. As illustrated, the application 860 comprises numerous functional components for implementing the features and operations of the exemplary embodiments. The application 860 of the computer 800 may represent various applications, computational units, logic, functional units, processes, operations, virtual entities, and/or modules in accordance with exemplary embodiments, but the application 860 is not meant to be a limitation.

The operating system 850 controls the execution of other computer programs, and provides scheduling, input-output control, file and data management, memory management, and communication control and related services. It is contemplated by the inventors that the application 860 for implementing exemplary embodiments may be applicable on all commercially available operating systems.

Application 860 may be a source program, executable program (object code), script, or any other entity comprising a set of instructions to be performed. When a source program, then the program is usually translated via a compiler (such as the compiler 840), assembler, interpreter, or the like, which may or may not be included within the memory 820, so as to operate properly in connection with the O/S 850. Furthermore, the application 860 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 870 may include input devices such as, for example but not limited to, a mouse, keyboard, scanner, microphone, camera, etc. Furthermore, the I/O devices 870 may also include output devices, for example but not limited to a printer, display, etc. Finally, the I/O devices 870 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 870 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at startup, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 860 and the O/S 850 are read, in whole or in part, by the processor 810, perhaps buffered within the processor 810, and then executed.

When the application 860 is implemented in software it should be noted that the application 860 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 860 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand various embodiments with various modifications are contemplated.

## Claims

1. A method for providing subject-specific information, the method comprising:
obtaining a plurality of clinical maps associated with a subject, each clinical map comprising graphical information for graphically representing clinical data relating to the subject;
obtaining clinical data relating to the subject, the clinical data comprising information describing a time and a value of a property of each of a plurality of clinical events; and
generating a subject-specific clinical model based on the plurality of clinical maps and the obtained clinical data, the subject-specific clinical model comprising a hierarchical representation of the plurality of clinical maps.

2. The method of claim 1, wherein the subject-specific clinical model is representative of an anatomical relationship between anatomical features of the subject.

3. The method of any preceding claim, wherein the clinical data further comprises information describing an anatomical feature associated with each of the plurality of clinical events,
and wherein the step of generating a subject-specific clinical model comprises defining a hierarchical representation of the plurality of clinical maps based on the information describing an anatomical feature associated with each of the plurality of clinical events.

4. The method of any preceding claim, wherein the step of generating a subject-specific clinical model comprises:
combining at least two of the plurality of clinical maps based on a blending function,
and wherein the blending function is representative of at least one of: a definition of an instance of a clinical model; a hierarchy of clinical models; and a hierarchy of clinical maps.

5. The method of any preceding claim, further comprising:
annotating the generated subject-specific clinical model based on the information describing a time of each of the plurality of clinical events.

6. A method for displaying subject-specific information, the method comprising:
obtaining a subject-specific clinical model comprising a hierarchical representation of a plurality of clinical maps and clinical data relating to the subject, the clinical data comprising information describing a time and a value of a property of each of a plurality of clinical events;
receiving an input signal representative of at least one of: a selected time-window; and a selected clinical map of the subject-specific clinical model;
generating a control signal for displaying clinical data relating to the subject based on the subject-specific clinical model and the received input signal; and
displaying clinical data relating to the subject in accordance with the generated control signal.

7. The method of claim 6, wherein the input signal is representative of a selected time-window,
and wherein the step of generating a control signal for displaying clinical data relating to the subject comprises generating a control signal for controlling the display of clinical data based on whether a time of a clinical event matches the selected time-window.

8. The method of claim 6 or 7, wherein the input signal is representative of a selected clinical map of the subject-specific clinical model,
and wherein the step of generating a control signal for displaying clinical data relating to the subject comprises generating a control signal for controlling the display of clinical data based on whether a value of a property of a clinical event relates to the selected clinical map.

9. The method of any of claims 6 to 8, wherein the step of generating a control signal is further based on a display position of the selected clinical map of the subject-specific clinical model.

10. A computer program product for providing or displaying subj ect-specific information, wherein the computer program product comprises a computer-readable storage medium having computer-readable program code embodied therewith, the computer-readable program code configured to perform all of the steps of any preceding claim.

11. A computer system comprising: a computer program product according to claim 10; and one or more processors adapted to perform a method according to any of claims 1 to 9 by execution of the computer-readable program code of said computer program product.

12. A system for providing subject-specific information, the system comprising:
an input interface adapted to obtain a plurality of clinical maps associated with a subject, each clinical map comprising graphical information for graphically representing clinical data relating to the subject, and to obtain clinical data relating to the subject, the clinical data comprising information describing a time and a value of a property of each of a plurality of clinical events; and
a processing module adapted to generate a subject-specific clinical model based on the plurality of clinical maps and the obtained clinical data, the subject-specific clinical model comprising a hierarchical representation of the plurality of clinical maps.

13. The system of claim 12, wherein the clinical data further comprises information describing an anatomical feature associated with each of the plurality of clinical events,
and wherein the processing module is adapted to define a hierarchical representation of the plurality of clinical maps based on the information describing an anatomical feature associated with each of the plurality of clinical events.

14. A system for displaying subject-specific information, the system comprising:
an interface adapted to obtain a subject-specific clinical model comprising a hierarchical representation of a plurality of clinical maps and clinical data relating to the subject, the clinical data comprising information describing a time and a value of a property of each of a plurality of clinical events;
an input signal interface adapted to receive an input signal representative of at least one of: a selected time-window; and a selected clinical map of the subject-specific clinical model;
an output interface adapted to generate a control signal for displaying clinical data relating to the subject based on the subject-specific clinical model and the received input signal; and
a display unit adapted to display clinical data relating to the subject in accordance with the generated control signal.

15. The system of claim 14, wherein the input signal is representative of a selected time-window,
and wherein the output interface is adapted to generate a control signal for controlling the display of clinical data based on whether a time of a clinical event matches the selected time-window.
